(19)

Europäisches
Patentamt
European
Patent Office
Office européen
des brevets

(11) **EP 2 502 555 A1**

(12) **EUROPEAN PATENT APPLICATION**

(43) Date of publication:
26.09.2012 Bulletin 2012/39

(21) Application number: 11159127.7

(22) Date of filing: 22.03.2011

(51) Int Cl.:
*A61B 5/022* (2006.01)    *A61B 5/0225* (2006.01)
*A61B 5/024* (2006.01)    *A61B 5/026* (2006.01)
*A61B 5/029* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Bmeye B.V.
1101 BE Amsterdam (NL)

(72) Inventor: Settels, Jacobus Jozef Gerardus Maria
1426 AR De Hoef (NL)

(74) Representative: Heselberger, Johannes
Bardehle Pagenberg
Partnerschaft
Patentanwälte, Rechtsanwälte
Prinzregentenplatz 7
81675 München (DE)

(54) **Non-invasive oxygen delivery measurement system and method**

(57)    A method of determining the oxygen delivery, comprising the steps of non-invasive measurement of the arterial blood pressure waveform, determination of cardiac output from a pulse wave analysis of said blood pressure waveform, non-invasive measurement of oxygen saturation and hemoglobin concentration and real time calculation of oxygen delivery based on the measured quantities.

**Fig. 1**

EP 2 502 555 A1

**Description**

**1. Technical Field**

The present invention relates to a system and method of measuring in real time and totally non-invasively, the physiological delivery of oxygen by the blood flow to the tissues of a patient.

**2. The Prior Art**

One of the challenges of clinicians is to obtain accurate information on the transport or delivery of oxygen to the tissues of a patient. This is important during many medical procedures, because the clinician needs to know and decide if and when to intervene with fluids, blood or pharmacological agents when the oxygen delivery to the tissues of a patient is not adequate in relation to the metabolic demand. The fundamental question is: is oxygen delivery adequate for the needs of the patient's tissues, and, if not, does the therapy applied result in a timely improvement of the transport or delivery of oxygen.

The continuous determination of oxygen delivery is important, because a sustained cardiovascular insufficiency with inadequate oxygen delivery to the patient's tissues may result in end-organ dysfunction and increased patient morbidity and mortality. Already in the 1980s, it was documented that surgery-associated reduction of global oxygen delivery (DO2) resulted in a surgery-induced oxygen delivery debt (Shoemaker et al, Crit Care Med 1988; 16: 1117-1120). The magnitude of this oxygen debt was uniquely associated with patient outcome. More recently, Gutierrez (Gutierrez et al, Lancet 1992;339: 195-199) demonstrated that targeted threshold values of DO2 were only effective at reducing long-term morbidity if applied before the development of end-organ failure. It is also important for evaluating stability of hemodynamic instable patients. Anesthesiologists and intensivists are usually well aware of the importance of this time factor, and sometimes refer to it as "the golden hour" during which the required corrections should be made.

At the present time, most clinicians rely on invasive pulmonary artery catheterization to directly measure the cardiac output, which relates to the blood flow, combined with the drawing of an arterial blood sample from an arterial line to directly measure the hemoglobin (Hb), i.e. the oxygen carrier concentration and its saturation with oxygen (Sa02).

The International Patent Application WO 03/092491 relates to a blood flow oxygen measurement system which is based on continuous wave ultrasound Doppler flow velocity data. Because of the lack of absolute cardiac output information, it focuses on relative output variables instead, which is inferior to an absolute measurement. As a means to provide oxygen saturation, pulse oximetry is mentioned. Relative tissue perfusion indexes are discussed based on combinations of oxygen saturation, velocity-time-integral (vti), heart rate (HR) and Doppler flow profile cross sectional area (XSA). However, ultrasound systems, apart from the lack of absolute accuracy, are strongly limited in clinical uses because of the required operator skills and the high susceptibility to motion artifacts which practically precludes any longer time monitoring.

The International Patent Application WO 96/39928 discloses a system for the real time prediction of a patient's mixed venous oxygen tension (PvO2) and cardiac output (CO). It uses the arterial oxygen content (Ca02) as determined by an invasive blood chemistry monitor and the patient's oxygen consumption as input value in the computation of PvO2. However, the system is partly invasive, and aims at the oxygen consumption and venous oxygen tension side rather than on the oxygen delivery side.

As a result, DO2 optimization strategies are not widely used to date in clinical patient care. It is the object of this invention, to provide a system and method for real time measurement of the oxygen delivery, allowing a fast and continuous monitoring of the relevant body functions, thereby overcoming the above mentioned problems of the prior art at least in part.

**3. Summary of the Invention**

This object is solved according to the present invention by a method of determining the oxygen delivery, comprising the steps of non-invasive measurement of the arterial blood pressure waveform, determination of cardiac output from a waveform analysis of said measured blood pressure waveform, non-invasive measurement of oxygen saturation and hemoglobin concentration, and real time calculation of oxygen delivery based on the measured quantities.

This approach is particularly advantageous since it solves for the first time the main problems underlying the present invention as it provides a totally non-invasive DO2 monitoring method which also provides the values in real time. The DO2 data is provided in a manner which fully synchronizes the variability inherently present in the underlying individual physiological parameters that together make up the DO2 result. In more detail, for calculating the oxygen delivery the cardiac output, the oxygen saturation and the hemoglobin concentration are required. An exact determination is only possible, if these parameters can be determined as synchronously, i.e. as much belonging together in time, as possible.

Also, there is no need any more for percutaneous instruments which would require additional safety measures and specially trained personal. In contrast, non-invasive sensors can easily be applied, e.g., by paramedics or emergency physicians. Therefore, it is easier to determine an appropriate therapy in shorter time. This will finally reduce the mortality of patients, when using the method according to the present invention in concert with proven therapeutic approaches that utilize its diagnostic power correctly (cf. Pinsky, Intensive Care Med 2004;30:1008-1010). In particular, the totally non-invasive measurement of DO2 makes it possible to also apply the method and system in low to medium risk patients, who are not considered at risk enough to warrant the use of invasive lines for monitoring, but in whom DO2 monitoring is considered useful, e.g., because of the presence of comorbidities such as diabetes, obesity, hypertension or smoking habits. The non-invasive measurement of a continuous plethysmographic waveform using pulse oximetry to provide a real time oxygen saturation (SpO2) as an estimate of Sa02 is applied, as known e.g., from the US Patents 4,653,498 and 7,530,955. The non-invasive real time oxygen carrier concentration (SpHb) measurement as an estimate of total blood Hb using pulse oximetry or CO pulse oximetry can be applied, as disclosed, e.g., by the International Patent Application WO 2006/118654.

In another aspect, the oxygen delivery is calculated by multiplying the cardiac output, oxygen saturation, hemoglobin concentration and a version of Hufner's constant.

Accordingly, by measuring these values in a non-invasive manner, the desired parameter, i.e., the oxygen delivery can be calculated by a simple multiplication: the total oxygen delivery (DO2) is determined by the oxygen content (Ca02), made up by the concentration of oxygen carriers, i.e. hemoglobin (Hb), and the saturation with oxygen of these carriers (SaO2)) and by the transport capacity, i.e. the blood flow or cardiac output (CO):

$$DO_2 = k * SaO_2 * Hb * CO \qquad\qquad \text{(equation 1)}$$

or, alternatively stated:

$$DO_2 = k * CaO_2 * CO \qquad\qquad \text{(equation 2)}$$

In particular, Hufner's constant (k) is chosen such that it takes into account the units for the cardiac output, oxygen saturation and the hemoglobin concentration.

The oxygen delivery (DO2) is expressed as the amount of oxygen (in ml/min) made available to the patient's tissues in one minute. This oxygen delivery can be normalized to the patient's body surface, which means that it is based on the cardiac index (CI) instead of the cardiac output (CO).

According to another aspect, the oxygen delivery parameter can be combined with a determination of oxygen consumption (VO2) to determine an oxygen extraction ratio which can be used as an indicator whether oxygen delivery is adequate in relation to metabolic demands of a patient. In more detail, the oxygen extraction ratio can be advantageously determined by dividing a measured or input oxygen consumption measure (VO2) by the oxygen delivery.

According to another aspect of the present invention, the arterial blood pressure waveform is measured by a finger arterial volume clamp method, in which the arterial volume is kept at a constant level by varying the pressure outside the artery in concordance with intra arterial blood pressure.

The volume clamp method allows for providing a continuous blood pressure waveform, relatively free of artifacts, compensated by hydrostatic pressure differences when the measured finger is not at heart level, which is also discussed in the EP 48060 and the EP 78090.

In another aspect, the measured finger arterial blood pressure waveform is reconstructed into a more central blood pressure waveform such as the brachial arterial pressure waveform.

The reconstruction of the brachial arterial blood pressure waveform from the finger arterial blood pressure waveform provides beat-to-beat systolic, diastolic, mean pressure and heart rate. Such reconstruction of the brachial arterial blood pressure waveform is also known, e.g., from Gizdulich et al, Cardiovasc Res 1997;33:689-705.

Moreover, analysis of the brachial arterial pressure waveform using pulse contour analysis (such as Nexfin CO-TREK) is possible, to provide beat to beat stroke volume and cardiac output. This is also discussed by the International Patent Application WO 2009/014420, which is incorporated herein by reference.

The primary goal of resuscitation is to increase DO2. Thus the primary functional question usually asked in the hemodynamically unstable patient is: will cardiac output (the primary variable) increase with fluid resuscitation (the primary therapeutic agent) and if so by how much? Physiologically, this equates to the question if the patient is fluid responsive. One of the methods currently used in assessing fluid responsiveness is to infuse a small bolus rapidly and evaluate the response by observing changes in arterial blood pressure, heart rate, cardiac output, blood lactate or other relevant parameters. A patient is considered a "responder" if there is an improvement. One of the disadvantages of this approach is that it is only positive in half of the hypotensive patients. A volume challenge given to "non-responder" may worsen or precipitate pulmonary edema and cause cardiac overload.

Recent developments provide alternative methods that mimic a transient volume challenge without any volume actually being administered, which obviates the risks in "non-responders". Positive pressure ventilation in anesthetized or sedated patients cyclically alters the pressure gradient for venous return, thereby inducing cyclic changes in cardiac stroke volume. When a fixed ventilatory tidal volume is used, the degree in variation in systolic pressure, pulse pressure and stroke volume reflects fluid responsiveness and the Stroke Volume Variation (SVV) and Pulse Pressure Variation (PPV) indexes can thus be used as additional guidance information in order to predict the direction and magnitude of the response of a patient to DO2 therapy by fluid resuscitation before the actual start of the therapy (Cannesson, J Cardiothor Vasc Anesthesia 2010;3:487-497). The interpretation of the DO2 measurement is supported by the totally non-invasive measurement of stroke volume variation and pulse pressure variation derived from a pulse wave analysis of said blood pressure waveform in a fully synchronized manner. The computation of a real time stroke volume variation and the pulse pressure variation by analysis of the blood pressure wave form and of the beat to beat stroke volume data can be applied, as disclosed, e.g., by Michard et al, Am J Resp Crit Care Med 2000;162:134-138.

In another aspect, the oxygen delivery parameter is combined with an analysis of respiratory variations in beat-to-beat data, so that information about the oxygen delivery as well as about the fluid responsiveness state of a patient is available.

From such an analysis of respiratory variations in beat-to-beat data such as for instance pulse pressure variation and/or stroke volume variation, information about the oxygen delivery as well as about the fluid responsiveness state of a patient is available.

According to another aspect, the pulse pressure variation and stroke volume variation are monitored using the arterial blood pressure waveform.

In yet another aspect, the arterial blood pressure is measured non-invasively with at least one sensor on a finger and wherein the oxygen saturation and the hemoglobin concentration are non-invasively measured with at least one multi-wavelength sensor. This sensor could be placed on another finger.

Similar to the above, determining these parameters without any percutaneous instruments allows for faster determination of the oxygen delivery and thus for an improved determination of the required therapy.

The present invention also relates to an apparatus for determining the oxygen delivery according to any of the above discussed aspects.

The method could easily be applied to an apparatus, e.g., for use by anaesthetists or paramedics in order to determine the oxygen delivery in an easy and fast way, thereby saving critical time, e.g., for determining the oxygen delivery in a patient's tissues.

Even if some embodiments have only been described in view of a method, it is to be understood that all embodiments can be carried out as an apparatus and vice versa.

## 4. Short Description of the Drawings

In the following aspects of the present invention are discussed with respect to the accompanying figures. These figures show:

Fig. 1:    shows a block diagram of a patient monitoring system according to several embodiments;

Fig. 2:    shows a block diagram according to several embodiments of the present invention;

Fig. 3:    shows several sensors for determining the numerical values in accordance with several embodiments; and

Fig. 4:    shows a possible illustration of numerical values in accordance with several embodiments.

## 5. Detailed Description

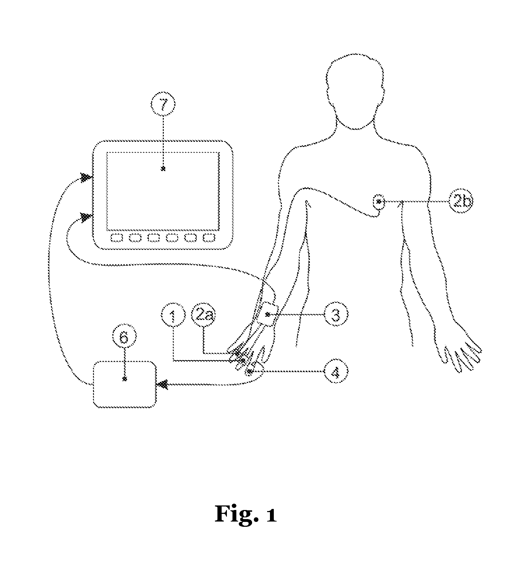

Fig. 1 illustrates a block diagram of an exemplary embodiment of a patient monitoring system using the disclosed methods. In an embodiment, the sensor 1 is placed on the middle phalanx of a patient's finger and is connected to a pre-processing unit 3 which measures the continuous finger arterial blood pressure waveform using a volume clamp method. Sensor 2a and 2b together with the tubing that connects them form a hydrostatic height measurement system, with one end 2a typically placed near the sensor 1 and the other end 2b placed at a reference level, typically heart level.

Unit **3** also performs the automatic compensation of the measured finger arterial blood pressure for any measured hydrostatic height differences between **2a** and **2b.**

Sensor **4** is placed on the finger tip of another finger, which is typically an adjacent finger but can also be the same finger as used for sensor **1**. It is connected to a pre-processing unit **6** which measures the continuous finger plethysmographic waveform and derives the real-time oxygen saturation (SpO2) and the real-time oxygen carrier concentration (SpHb), using a pulse oximetry method.

Pre-processing units **3** and **6** are connected to a patient monitoring system **7**, which together perform the following tasks in real time:

- Collection and storage of all information collected from units **3** and **6**;
- Reconstruction of a brachial arterial blood pressure waveform from the finger arterial blood pressure waveform, to provide beat-to-beat systolic, diastolic, mean pressure and heart rate;
- Analysis of the brachial arterial pressure waveform using pulse contour analysis, to provide beat to beat stroke volume and cardiac output;
- Computation of a real-time stroke volume variation (SW) and pulse pressure variation (PPV) by analysis of the blood pressure waveform - for PPV - and of the beat to beat stroke volume data - for SW;
- Computation of the oxygen delivery (DO2) in absolute values (such as ml/min) or in relative values (such as %) using the fully synchronized in time data available from the steps above, using above said equation 1 or equation 2;
- Real-time display of numerical values and trends of DO2, SVV and PPV in combination with other relevant hemodynamic parameters.

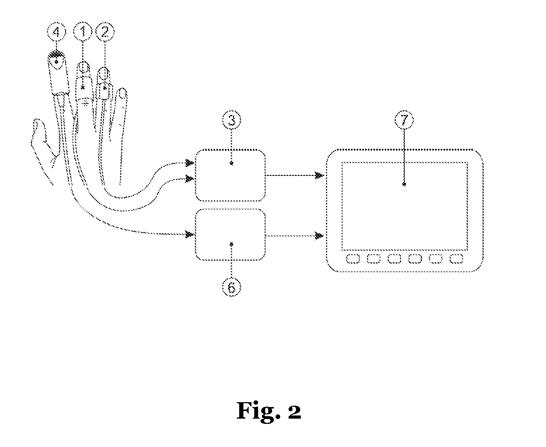

Fig. **2** shows an example of a configuration for performing the measurements according to some embodiments of the present invention. Sensor **4** is connected with pre-processing unit **6** which measures and derives the real-time oxygen saturation (SpO2) and the real-time oxygen carrier concentration (SpHb), using the pulse oximetry method. Sensors **1** and **2** are connected with pre-processing unit **3** which measures the continuous finger arterial blood pressure waveform using the volume clamp method. The pre-processing units **3** and **6** are connected with patient monitoring system **7** which together can perform the functions described above.

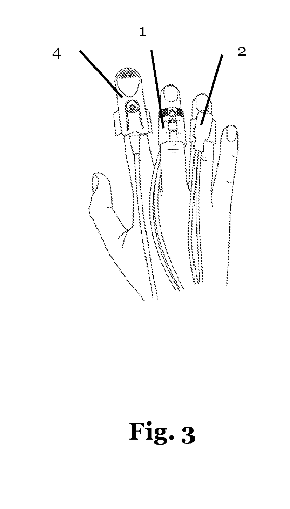

Fig. **3** illustrates the sensors again in more detail. Therein, sensor **4** is used for the continuous finger plethysmographic waveform measurement and sensors **1** and **2** measure the continuous finger arterial blood pressure waveform using the volume clamp method.

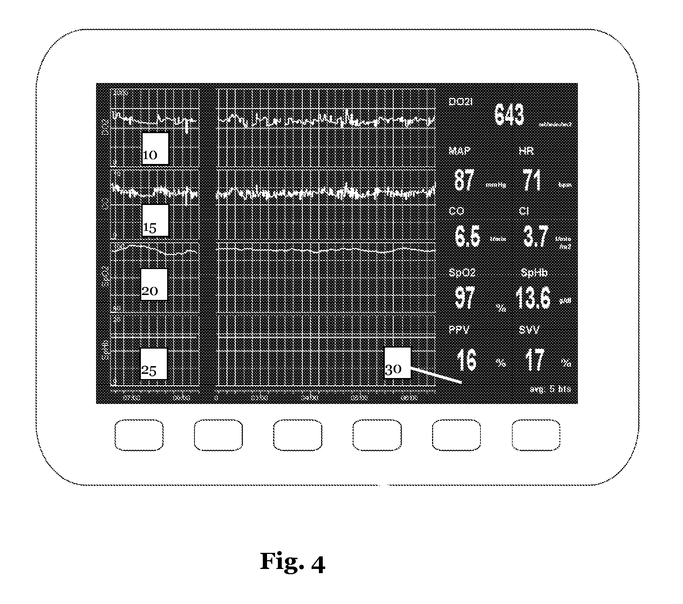

Fig. **4** shows an exemplary display of the patient monitoring system 7. In this embodiment, the system may display trend information of the oxygen delivery **10,** the cardiac output **15**, the arterial oxygen saturation **20** and the total hemoglobin **25** in the left part of the figure. Furthermore, in the right part **30** of the display, real time measurements of the parameters are shown.

In more detail, in an embodiment, the display advantageously displays one or more of the following: mean arterial pressure (MAP), heart rate (HR), cardiac output (CO), cardiac index (CI), oxygen delivery (DO2), oxygen delivery index (DO2I), arterial oxygen saturation (SpO2), total hemoglobin (SpHb), stroke volume variation (SVV), pulse pressure variation (PPV), or similar, as shown in the right part **30** of the display.

Furthermore, the display may show one or more parameters in the form of a relative or absolute deviation from a baseline value as can be established from previous parameter values. This can be achieved, e.g., by averaging over a certain time window.

Accordingly, the present invention includes a multi-parameter patient monitor 7 capable of determining one or more of the foregoing parameters, other than or in addition to the blood pressure waveform and derived beat-to-beat variables like systolic and diastolic pressures.

In an embodiment, the display of a non-invasive DO2 monitor advantageously includes a plurality of display modes enabling a plurality of data to be displayed, more than the available physical display area. A user may cycle different parameter display values and trend modes, and the display may use different parameters as color-coded.

Moreover, parameter trends may be displayed using the same or different color codings, especially when multiple trends are displayed on one or more display panels.

The embodiments shown in the figures merely represent examples. For instance, any other number and / or combination of sensors could be used for performing the measurements.

**Claims**

1. Method of determining the oxygen delivery to a patient's tissues, comprising the steps of:

   non-invasive measurement of the arterial blood pressure waveform;

determination of cardiac output from a pulse wave analysis of said blood pressure waveform;
non-invasive measurement of oxygen saturation and hemoglobin concentration; and
real time calculation of oxygen delivery.

2. Method according to claim 1, wherein the oxygen delivery is calculated by multiplying the Cardiac Output, Oxygen Saturation, hemoglobin concentration and a version of Hufner's constant.

3. Method according to any of the preceding claims, wherein the arterial blood pressure waveform is measured by a finger arterial volume clamp method, in which the arterial volume is kept at a constant level by varying the pressure outside the artery in concordance with intra arterial blood pressure.

4. Method according to any of the preceding claims, wherein the measured finger arterial blood pressure waveform is reconstructed into a more central blood pressure waveform such as the brachial arterial pressure waveform.

5. Method according to any of the preceding claims, wherein the oxygen delivery parameter is combined with an analysis of respiratory variations in beat-to-beat data, so that information about the oxygen delivery as well as about the fluid responsiveness state of a patient is available.

6. Method according to any of the preceding claims, wherein the oxygen delivery parameter is combined with a determination of oxygen consumption (VO2) to determine an oxygen extraction ratio which can be used as an indicator whether oxygen delivery is adequate in relation to metabolic demands of a patient.

7. Method according to any of the preceding claims, wherein pulse pressure variation and stroke volume variation are monitored using the arterial blood pressure waveform.

8. Method according to any of the preceding claims, wherein the arterial blood pressure is measured non-invasively with at least one sensor on a finger and wherein the oxygen saturation and the hemoglobin is non-invasively measured with at least one multi-wavelength sensor on another finger.

9. Apparatus for determining the oxygen delivery, comprising:

means for non-invasive measurement of the arterial blood pressure waveform;
means for determination of cardiac output from a pulse wave analysis of said blood pressure waveform;
means for non-invasive measurement of oxygen saturation and hemoglobin comprising at least one multi-wavelength sensor;
and
means for real time calculation of oxygen delivery.

10. Apparatus according to claim 9, wherein the oxygen delivery is calculated by multiplying the Cardiac Output, Oxygen Saturation, hemoglobin concentration and a conversion constant.

11. Apparatus according to any of the claims 9 or 10, further comprising:

means for measuring the arterial blood pressure waveform by a finger arterial volume clamp method, in which the arterial volume is kept at a constant level by varying the pressure outside the artery in concordance with intra arterial blood pressure.

12. Apparatus according to any of the claims 9 to 11, wherein the measured finger arterial blood pressure waveform is reconstructed into a more central blood pressure waveform such as the brachial arterial pressure waveform.

13. Apparatus according to any of the claims 9 to 12, further comprising means for combining the oxygen delivery parameter with an analysis of respiratory variations in beat-to-beat data, so that information about the oxygen delivery as well as about the fluid state of a patient is available.

14. Apparatus according to any of the claims 9 to 13, wherein the oxygen delivery parameter is combined with a determination of oxygen consumption (VO2) to determine an oxygen extraction ratio which can be used as an indicator whether oxygen delivery is adequate in relation to metabolic demands of a patient.

**15.** Apparatus according to any of the claims 9 to 14, wherein pulse pressure variation and stroke volume variation are monitored using the arterial blood pressure waveform.

Fig. 1

**Fig. 2**

**Fig. 3**

**Fig. 4**

Europäisches Patentamt
European Patent Office
Office européen des brevets

# EUROPEAN SEARCH REPORT

Application Number

EP 11 15 9127

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2006 051561 A1 (WEINMANN G GERAETE MED [DE]) 24 May 2007 (2007-05-24) | 1,2, 7-10,15 | INV.<br>A61B5/022 |
| Y | * the whole document * | 3-6,11, 12,14 | A61B5/0225<br>A61B5/024<br>A61B5/026 |
| X | US 2003/135124 A1 (RUSSELL TED W [US]) 17 July 2003 (2003-07-17) | 9,10,13, 15 | A61B5/029 |
| Y | * paragraphs [0067] - [0076], [0099] - [0100], [0105], [0125] - [0126], [0132] - [0148], [0154] - [0172] *<br>* paragraphs [0201] - [0217]; claims; figures * | 11,12,14 | |
| Y | US 6 743 172 B1 (BLIKE GEORGE T [US]) 1 June 2004 (2004-06-01)<br>* column 2, lines 41-65 *<br>* column 3, lines 23-35 *<br>* column 4, lines 1-26 *<br>* column 5, line 33 - column 7, line 14 *<br>* column 8, lines 43-49 *<br>* column 10, line 48 - column 20, line 35 *<br>* column 26, lines 1-52 * | 3,5,6,11 | |
| Y | & US 5 183 051 A (KRAIDIN JONATHAN [US] ET AL) 2 February 1993 (1993-02-02)<br>* the whole document * | 3,5,6, 11,14 | |
| Y | BOGERT LYSANDER W J ET AL: "Non-invasive pulsatile arterial pressure and stroke volume changes from the human finger", EXPERIMENTAL PHYSIOLOGY, CAMBRIDGE UNIVERSITY PRESS, CAMBRIDGE, GB, vol. 90, no. 4, 1 July 2005 (2005-07-01), pages 437-446, XP002441094, ISSN: 0958-0670, DOI: 10.1113/EXPPHYSIOL.2005.030262<br>* the whole document * | 3-5,11, 12 | |

TECHNICAL FIELDS SEARCHED (IPC)

A61B

The present search report has been drawn up for all claims

| Place of search | Date of completion of the search | Examiner |
|---|---|---|
| The Hague | 7 September 2011 | Crisan, Carmen-Clara |

**EP 2 502 555 A1**

## ANNEX TO THE EUROPEAN SEARCH REPORT
## ON EUROPEAN PATENT APPLICATION NO.

EP 11 15 9127

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2011

| Patent document cited in search report | Publication date | Patent family member(s) | Publication date |
|---|---|---|---|
| DE 102006051561 A1 | 24-05-2007 | NONE | |
| US 2003135124 A1 | 17-07-2003 | NONE | |
| US 6743172 B1 | 01-06-2004 | NONE | |

EPO FORM P0459

For more details about this annex : see Official Journal of the European Patent Office, No. 12/82

13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03092491 A
- WO 9639928 A
- US 4653498 A
- US 7530955 A
- WO 2006118654 A
- EP 48060 A
- EP 78090 A
- WO 2009014420 A

**Non-patent literature cited in the description**

- **SHOEMAKER et al.** *Crit Care Med,* 1988, vol. 16, 1117-1120
- **GUTIERREZ et al.** *Lancet,* 1992, vol. 339, 195-199
- **PINSKY.** *Intensive Care Med,* 2004, vol. 30, 1008-1010
- **GIZDULICH et al.** *Cardiovasc Res,* 1997, vol. 33, 689-705
- **CANNESSON.** *J Cardiothor Vasc Anesthesia,* 2010, vol. 3, 487-497
- **MICHARD et al.** *Am J Resp Crit Care Med,* 2000, vol. 162, 134-138